# EUROPÄISCHE PATENTSCHRIFT

(11) **EP 2 337 541 B1**
(45) Veröffentlichungstag und Bekanntmachung des Hinweises auf die Patenterteilung: **07.03.2012**
(21) Anmeldenummer: 09778356.7
(22) Anmeldetag: 05.09.2009
(51) Int. Cl.: A61F 13/15

(54) **ABSORBIERENDE INKONTINENZWEGWERFWINDEL**
ABSORBING DISPOSABLE INCONTINENCE DIAPER
COUCHE D'INCONTINENCE ABSORBANTE JETABLE

(30) Priorität: 09.09.2008 DE 102008046358
(43) Veröffentlichungstag der Anmeldung: 29.06.2011
(73) Patentinhaber: Paul Hartmann AG, 89522 Heidenheim (DE)
(72) Erfinder: OSTERTAG, Wolfgang, 89547 Gerstetten (DE)
(86) Internationale Anmeldenummer: PCT/EP2009/006460
(87) Internationale Veröffentlichungsnummer: WO 2010/028786

(56) Entgegenhaltungen:
- EP-A1- 1 719 484
- EP-A1- 2 020 215
- EP-A2- 1 269 949
- WO-A1-03/082168
- WO-A1-03/094815
- WO-A1-2007/042084

## Beschreibung

Die vorliegende Erfindung betrifft die Herstellung einer absorbierenden Inkontinenzwegwerfwindel des offenen Typs mit einem Hauptteil umfassend einen Vorderbereich, einen Rückenbereich und einen in Längsrichtung dazwischen liegenden, zwischen den Beinen eines Benutzers zu liegen kommenden Schrittbereich, wobei der Hauptteil einen Saugkörper umfasst, und mit beidseits an den Rückenbereich angefügten hinteren Seitenabschnitten und beidseits an den Vorderbereich angefügten vorderen Seitenabschnitten. Die Inkontinenzwegwerfwindel ist für Erwachsene vorgesehen und zum einmaligen Gebrauch bestimmt.

Derartige Inkontinenzwegwerfwindeln sind beispielsweise aus DE102005048868A1 und WO03/082168 bekannt, wobei die WO03/082168 bereits lehrt, die Seitenabschnitte nach dem Anfügen an den Hauptteil durch Bildung von Materialaus- sparungen mit konturierten Beinöffnungen zu versehen. Die DE102005048868A1 offenbart auch bereits, die Seitenabschnitte unterschiedlich auszugestalten, nämlich die hinteren Seitenabschnitte mit einer höheren Dehnbarkeit zu versehen als die vorderen Seitenabschnitte.

Bei derartigen Inkontinenzwegwerfwindeln sind die erwähnten Seitenabschnitte häufig aus einem anderen Material gebildet als der Hauptteil. Beispielsweise können die Seitenabschnitte, die häufig auch als "Ohren" der Inkontinenzwegwerfwindel bezeichnet werden, atmungsaktiv, insbesondere luft- und wasserdampfdurchlässig ausgebildet werden, wohingegen der Hauptteil, der häufig auch als Chassis bezeichnet wird, flüssigkeitsundurchlässig ausgeführt sein kann. Zum Schließen der Inkontinenzwegwerfwindel am Benutzer werden die vorzugsweise unlösbar am Rückenbereich angefügten Seitenabschnitte nach vorne, insbesondere bis auf die Bauchseite des Benutzers geschlagen und mittels Verschlusselementen dort entweder mit der Außenseite des Vorderbereichs des Hauptteils oder mit der Außenseite der Seitenabschnitte des Vorderbereichs in überlappender Konfiguration lösbar verbunden.

Wie in DE102005048868A1 dargestellt, weisen sowohl der Hauptteil als auch die Seitenabschnitte derartiger Inkontinenzwegwerfwindeln jeweils eine rechteckförmige Gestalt auf. Auch die DE102004021353A1 offenbart eine derartige Inkontinenzwegwerfwindel. Die DE102004021353A1 lehrt außerdem, die Seitenabschnitte vorzufalten und die Vorfaltung durch eine lösbare Fixierung vor Gebrauch festzulegen. An schnelllaufenden Windelmaschinen erfolgt eine Vorfaltung der Seitenabschnitte bevorzugt noch vor der Fixierung der Seitenabschnitte an dem Windelhauptteil, es werden also bevorzugt bereits vorgefaltete und in dieser Konfiguration vorfixierte Seitenabschnitte an den Windelhauptteil angefügt. Wenngleich sich diese Inkontinenzwindel auch bei hohen Geschwindigkeiten und damit sehr wirtschaftlich fertigen lässt und die vorgefalteten Seitenabschnitte sich bevorzugt mit einem Zug öffnen lassen, erweist sich der Tragekomfort der vorbekannten Inkontinenzwegwerfwindel jedoch als unbefriedigend.

Zur Lösung dieses Problems wurde mit der DE102007056126 (noch unveröffentlicht) bereits vorgeschlagen, die Seitenabschnitte zur Bildung von Beinöffnungsbereichen zumindest auf ihren dem Schrittbereich zugewandten Rändern schräg zur Längsrichtung verlaufend oder kurvenförmig auszubilden und den Hauptteil zumindest im Schrittbereich sanduhrförmig auszubilden, wobei der schräge oder kurvenförmige Verlauf der Seitenabschnitte und die sanduhrförmige Konturierung des Hauptteils an beiden Längsseiten der Inkontinenzwegwerfwindel durch je einen einzigen, die Seitenabschnitte und den Hauptteil erfassenden Abtrennvorgang gebildet sind. Der die Seitenabschnitte und den Hauptteil erfassende Abtrennvorgang erfolgt nach dieser Idee also zu einem Zeitpunkt, zu dem die vorderen und hinteren Seitenabschnitte bereits an den Hauptteil angefügt sind. Hierdurch ist sichergestellt, dass die Beinöffnungskonturierung der Seitenabschnitte sich gleichsam stetig im Schrittbereich des Hauptteils fortsetzt, um dort die sanduhrförmige Konturierung des Hauptteils zu bilden.

Die verfahrenstechnischen Herausforderungen zur Fertigung derartiger Inkontinenzartikel in schnelllaufenden Windelmaschinen sind allerdings signifikant, da die Inkontinenzartikel mit den weit ausladenden Seitenabschnitten nur schwer stabil durch die schnelllaufende Windelmaschine förderbar sind und die erforderliche Präzision des die Beinöffnungsbereiche erzeugenden Abtrennvorgangs nur schwer gewährleistbar ist.

Zur Lösung dieses Problems wird ein Verfahren zur Herstellung von Inkontinenzwindeln des offenen Typs mit einem Hauptteil umfassend einen Vorderbereich mit vorderen seitlichen Längsrändern, einen Rückenbereich mit hinteren seitlichen Längsrändern und einem in Längsrichtung dazwischen liegenden, zwischen den Beinen eines Benutzers zu liegen kommenden Schrittbereich, wobei der Hauptteil einen Saugkörper umfasst, und mit beidseits an den Rückenbereich angefügten hinteren Seitenabschnitten und beidseits an den Vorderbereich angefügten vorderen Seitenabschnitten, welche sich in Querrichtung über die seitlichen vorderen und hinteren Längsränder des Hauptteils hinaus erstrecken und den Vorderbereich und den Rückenbereich im angelegten Zustand der Inkontinenzwegwerfwindel miteinander verbinden, wobei zur Bildung von Beinausschnittbereichen die Seitenabschnitte zumindest an den dem Schrittbereich zugewandten Rändern schräg zur Längsrichtung verlaufend oder kurvenförmig ausgebildet sind und der Hauptteil zumindest im Schrittbereich sanduhrförmig ausgebildet ist, vorgeschlagen, das folgende Verfahrensschritte umfasst:

Zuführen einer endlosen Flachmaterialbahn in einer Längsrichtung zur Herstellung von Materialabschnitten für die Bildung der Seitenabschnitte,

Bildung von ersten Materialaussparungen entlang einer ersten Schnittlinie an einem Seitenrand der Flachmaterialbahn zur Erzeugung von konturierten Ausschnitten in der Flachmaterialbahn, wobei eine gedachte Parallele zur Längsrichtung, welche an den Punkt P der maximalen Erstreckung des Ausschnittes quer zur Längsrichtung angelegt ist, einen äußeren Teilbereich und einen inneren Teilbereich der Flachmaterialbahn definiert

Falten der Flachmaterialbahn um mindestens eine in Längsrichtung verlaufende erste Faltlinie, wobei die Faltlinie innerhalb des äußeren Teilbereiches verläuft,

Abtrennen von Längsabschnitten der gefalteten Flachmaterialbahn zur jeweiligen Bildung von Materialabschnitten,

unlösbares Fixieren der Materialabschnitte an einem jeweiligen Längsrand einer Windelhauptteilbahn zur Bildung von Seitenabschnitten,

Bildung von zweiten Materialaussparungen entlang einer zweiten Schnittlinie, wobei die zweite Schnittlinie die Seitenabschnitte und einen jeweiligen Seitenrand der Windelhauptteilbahn erfasst, und wobei die zweite Schnittlinie die erste Schnittlinie kreuzt, derart dass sich die zweite Schnittlinie nicht durch die erste Faltlinie der vorderen und hinteren Seitenabschnitte erstreckt.

Die die späteren Seitenabschnitte bildende Flachmaterialbahn wird also zunächst mit ersten Materialaussparungen versehen, welche die Kontur von äußeren Teilabschnitten der späteren Beinöffnungsbereiche vorgeben. Anschließend wird die Flachmaterialbahn mindestens ein Mal gefaltet, so dass nach dem Abtrennen von Längsabschnitten bereits vorgefaltete Materialabschnitte an der Windelhauptteilbahn fixiert werden. Erst anschließend, also nach dem Fixieren der Materialabschnitte an der Windelhauptteilbahn, erfolgt der zweite Schnitt zur Erzeugung der zweiten Materialaussparungen. Wenn dieser zweite Schnitt zwar die erste Schnittlinie kreuzt, aber sich nicht durch die erste Faltlinie erstreckt, also gleichsam unterhalb der ersten Faltlinie ansetzt, kann sicher vermieden werden, dass der Schnitt durch Faltabschnitte und Faltlinien der Seitenabschnitte hindurchgeführt wird. Durch die vorgefaltete Konfiguration der Materialabschnitte kann die noch unfertige Windelbahn in der schnelllaufenden Windelmaschine sicher und stabil geführt und weiter präzise maschinell bearbeitet werden. Das ist insbesondere darauf zurückzuführen, dass die maximale Breite B1 der zu führenden Windelbahn im Bereich der Seitenabschnitte, insbesondere im Verhältnis zur minimalen Breite B2 der Windelbahn im Schrittbereich vor der Bildung der zweiten Materialaussparungen deutlich reduziert ist, so dass die Windelbahn besser unter Zugspannung geführt werden kann.

Vorzugsweise wird die Hauptteilbahn mit einer Geschwindigkeit v1 von 100-700 m/min, insbesondere von 120-550 m/min weiter insbesondere von 130-450 m/min in der Längsrichtung gefördert.

Dieses Breitenverhältnis B1/B2 beträgt vorzugsweise mehr als 1,1 jedoch höchstens 2, insbesondere höchstens 1,8, weiter insbesondere höchstens 1,7, weiter insbesondere höchstens 1,6.

Vorzugsweise erstreckt sich die zweite Schnittlinie ausgehend von einem Punkt A auf der ersten Schnittlinie eines hinteren Seitenabschnittes in einer Kurve nach innen in Richtung Schrittbereich zunächst bis zu einem Punkt B eines jeweiligen hinteren Seitenrandes des Hauptteils und dann in den Hauptteil hinein, wobei der Punkt A in Querrichtung nach innen von der ersten Faltlinie beabstandet ist. Nachfolgend erstreckt sich die Schnittlinie kontinuierlich weiter durch den Schrittbereich des Hauptteils und daran anschließend in einer Kurve nach außen durch einen Punkt C des vorderen Seitenrandes des Hauptteils und schließlich bis zu einem Punkt D auf der ersten Schnittlinie der vorderen Seitenabschnitte, wobei der Punkt D in Querrichtung nach innen von der ersten Faltlinie der vorderen Seitenabschnitte beabstandet ist.

Es hat sich als besonderes vorteilhaft herausgestellt, wenn die Punkte A und D um 2-60 mm, insbesondere um 3-50 mm, weiter insbesondere um 4-40 mm, vorzugsweise höchstens um 30 mm, weiter vorzugsweise höchstens um 20 mm, weiter insbesondere höchstens um 10 mm und weiter insbesondere höchstens um 5 mm in Querrichtung nach innen von der jeweiligen ersten Faltlinie beabstandet sind.

Vorzugsweise beträgt die Breite der Seitenabschnitte in ausgefalteter Konfiguration, also deren Erstreckung über den Seitenrand des Windelhauptteils hinaus 10-40 cm, insbesondere 12-30 cm, weiter insbesondere 13-25 cm. Vorzugsweise weisen die vorderen Seitenabschnitte dabei die gleiche Breite auf wie die hinteren Seitenabschnitte.

Angesichts dieser großen Abmessungen hat es sich als besonders vorteilhaft herausgestellt, wenn die Flachmaterialbahn um eine zweite in Längsrichtung verlaufende Faltlinie, insbesondere z-förmig auf sich selbst gefaltet wird, wobei die zweite Faltlinie näher zum Seitenrand angeordnet ist als die erste Faltlinie. Besonders bevorzugt wird die Flachmaterialbahn um eine dritte in Längsrichtung verlaufende Faltlinie, insbesondere w-förmig auf sich selbst gefaltet, wobei die dritte Faltlinie näher zum Seitenrand angeordnet ist als die zweite Faltlinie. Die erste Faltlinie ist also diejenige Faltlinie, deren Abstand zum Hauptteil in Querrichtung betrachtet am geringsten ist. Nach einer bevorzugten Ausführungsform sind die aufeinander gefalteten Teilabschnitte der Seitenabschnitte lösbar fixiert, insbesondere durch Ultraschallschweißpunkte.

Die in der Längsrichtung geförderte Hauptteilbahn umfasst vorzugsweise ein Vliesstoffmaterial und/oder ein Saugkörpermaterial und/oder ein Backsheetmaterial. Bei dem Backsheetmaterial kann es sich insbesondere um ein Folienmaterial oder ein flüssigkeitsundurchlässiges Vliesmaterial oder ein Vlies-Folienlaminat handeln.

In einer ersten Variante des erfindungsgemäßen Verfahrens wird der Hauptteilbahn eine einzige Seitenabschnitte bildende Flachmaterialbahn zugeführt. Die von dieser Bahn abgetrennten Materialabschnitte bilden solchenfalls linke und rechte sowie vordere und hintere Seitenabschnitte des Inkontinenzartikels.

Nach einer zweiten Variante werden der Windelhauptteilbahn zwei Seitenabschnitte bildenden Flachmaterialbahnen zugeführt. Vorzugsweise bildet solchenfalls die eine Flachmaterialbahn linke und die andere rechte Seitenabschnitte. Denkbar wäre auch, dass die eine Flachmaterialbahn vordere und die andere hintere Windelseitenabschnitte bildet. Solchenfalls wäre es denkbar und vorteilhaft, wenn die Flachmaterialbahnen sich hinsichtlich mindestens einer Eigenschaft unterscheiden.

Vorzugsweise ist die Flachmaterialbahn doppelnutzig ausgeführt, derart dass aus einem Längsabschnitt der Flachmaterialbahn zwei Materialabschnitte hervorgehen, wobei die ersten Materialaussparungen und das Falten der Flachmaterialbahn beidseits also an einem ersten und an einem zweiten Längsrand der Flachmaterialbahn erfolgt und die Flachmaterialbahn in Längsrichtung geteilt wird. Solchenfalls wird die Flachmaterialbahn vorzugsweise nach der Bildung der ersten Materialaussparungen und nach dem Falten der Flachmaterialbahn in Längsrichtung geteilt. Weiterhin vorzugsweise werden vor dem Teilen der Flachmaterialbahn Verschlussmittel auf die Flachmaterialbahn aufgebracht.

Die von der Flachmaterialbahn abgetrennten Seitenabschnitte bildenden Materialabschnitte können jeweils einen einzigen vorderen und/oder hinteren Windelseitenabschnitt bilden. Vorzugsweise jedoch bildet ein Materialabschnitt zwei Windelseitenabschnitte. In besonderes bevorzugter Ausführungsform wird hierbei ein zwei Seitenabschnitte bildender Materialabschnitt an einer noch endlosen in der Längsrichtung geförderten Hauptteilbahn befestigt. Nach einer bevorzugten Ausführung dieser Verfahrensvariante wird zur Bildung von vereinzelten Inkontinenzwegwerfwindeln die Hauptteilbahn quer zur Längsachse durch die Abschnitte hindurch getrennt, das heißt, dass erst mit der Vereinzelung der Inkontinenzartikel eine Trennung des Materialabschnittes in zwei Seitenabschnitte erfolgt.

Es hat sich als vorteilhaft erwiesen, die Inkontinenzwegwerfwindeln hierbei derart zu fertigen, dass bei in der Längsrichtung aufeinander folgend geförderten Inkontinenzwegwerfwindeln der Rückenbereich der einen Inkontinenzwegwerfwindel an den Rückenbereich der anderen Inkontinenzwegwerfwindel anschließt und der Vorderbereich der einen Inkontinenzwegwerfwindel an den Vorderbereich der anderen Inkontinenzwegwerfwindel anschließt. Denkbar und vorteilhaft wäre auch, dass der Rückenbereich der einen Inkontinenzwegwerfwindel sich an den Vorderbereich der anderen Inkontinenzwegwerfwindel anschließt. Nach diesen beiden Verfahrensvarianten bildet jedenfalls je ein quer zur Längsrichtung von der Flachmaterialbahn abgetrennter Abschnitt, Seitenabschnitte von zwei aufeinander folgend geförderten Inkontinenzwegwerfwindeln.

Die Konturen der Materialaussparungen können gerade, insbesondere schräg zur Längsrichtung der Inkontinenzwegwerfwindel verlaufende Abschnitte und/oder kurvenförmige Abschnitte umfassen. In einer bevorzugten Ausführungsform weist die Kontur der Materialaussparungen ausschließlich kurvenförmige Abschnitte auf. Der minimale Kurvenradius beträgt hierbei vorzugsweise mindestens 5mm, besonders bevorzugt mindestens 10mm. Vorzugsweise umfasst die Kontur der Beinöffnungsbereiche kurvenförmige Abschnitte mit unterschiedlichem Kurvenradius.

Es hat sich weiter als vorteilhaft erwiesen, die vorderen und/oder hinteren Seitenabschnitte aus einem Vliesstoffmaterial zu bilden. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können. Um zu verhindern, dass beim Lösen der mechanischen Verschlusshilfen Fasern aus dem Vliesverbund herausgerissen werden, ist vorteilhaft, die Vliesstoffkomponente mit einem Prägemuster zu versehen, vermittels dessen vorzugsweise alle Fasern der Vlieskomponente gebunden sind. Vorteilhaft ist solchenfalls insbesondere ein Thermoprägemuster, das insbesondere vorteilhaft durch Kalandern des Vliesstoffes unter Zuführung von thermischer Energie erzeugt wird.

Des Weiteren erweist es sich als vorteilhaft, dass seitlich neben den Längsrändern des Saugkörpers erste elastische Elemente mit einer Komponente in Längsrichtung an den Hauptteil angefügt sind. Diese elastischen Elemente können exakt, das heißt geradlinig in Längsrichtung verlaufen oder besonders vorteilhaft auch einer gewissen Konturierung entlang der Beinöffnungen folgend vorgesehen werden. Die elastischen Elemente nehmen solchenfalls einen gekrümmten Verlauf entlang der Beinöffnung. In besonderer Weiterbildung dieses Erfindungsgedankens ist vorgesehen, dass die elastischen Elemente sich nicht in die Seitenabschnitte hinein erstrecken, sondern auf eine Positionierung innerhalb des Hauptteils limitiert sind. Des Weiteren können in der ersten Längsrichtung erstreckte zweite elastische Elemente, insbesondere in Form von so genannten und an sich zum Beispiel auch aus EP0263720A1 bekannten aufstehenden Cuff-Elementen, an die Windelhauptteilbahn angefügt werden. Diese vorzugsweise aufstehenden zweiten elastischen Elemente flankieren gewissermaßen ein Zentrum des Windelhauptteils oder Saugkörpers; sie können im Bereich der Saugkörperränder, innerhalb der Saugkörperränder oder außerhalb der Saugkörperränder vorgesehen werden. Sie bilden einen Seitenauslaufschutz der Inkontinenzwegwerfwindel.

In einer weiteren vorteilhaften Weiterbildung der Erfindung weisen die Seitenabschnitte eine Innenseite und eine Außenseite auf, wobei die hinteren Seitenabschnitte Verschlussmittel mit insbesondere mechanischen Verschlusshilfen aufweisen und wobei die Verschlussmittel zum bestimmungsgemäßen Festlegen der Inkontinenzwegwerfwindel an den Körper eines Menschen zumindest bereichsweise sowohl an der Außenseite der hinteren Seitenabschnitte als auch an der Außenseite der vorderen Seitenabschnitte lösbar festlegbar sind.
In Weiterbildung dieses Erfindungsgedankens ist vorgesehen, dass die Verschlussmittel zum bestimmungsgemäßen Festlegen der Inkontinenzwegwerfwindel an den Körper eines Menschen zumindest bereichsweise sowohl an der Außenseite des Hauptteils als auch an der Außenseite der vorderen Seitenabschnitte lösbar festlegbar sind, wobei die Haltekräfte zwischen den Verschlussmitteln und der Außenseite der vorderen Seitenabschnitte vorzugsweise größer sind als die Haltekräfte zwischen den Verschlussmitteln und der Außenseite des Hauptteils. Auch dies veranlasst den Benutzer in der Mehrzahl der Fälle, die Verschlussmittel an den vorderen Seitenabschnitten festzulegen.

Die Außenseite des Hauptteils der Inkontinenzwegwerfwindel wird vorzugsweise zumindest bereichsweise, insbesondere aber vollflächig durch einen Vliesstoff gebildet. Dies vermittelt der Inkontinenzwegwerfwindel einen textilähnlichen Eindruck. Solchenfalls ist es vorteilhaft, das Backsheet des Hauptteils aus einem Vliesfolienlaminat zu bilden, wobei die Vlieslage außen und die Folienlage innen zum Saugkörper gerichtet zu liegen kommt, so dass die Vlieslage die Außenseite des Hauptteils bildet. Damit ist zum einem die Flüssigkeitsundurchlässigkeit des Hauptteils sichergestellt und zum anderen der hautfreundliche Charakter der Windel gesichert. Die Folienlage dieses Vliesfolienlaminates ist dann vorzugsweise aus einer ein- oder mehrschichtigen flüssigkeitsundurchlässigen, vorzugsweise aber gleichwohl atmungsaktiven Folie gebildet, wobei die Atmungsaktivität der vorderen und/oder der hinteren Seitenabschnitte vorzugsweise größer ist als die Atmungsaktivität des das Backsheet der Inkontinenzwegwerfwindel bildenden Vliesfolienlaminates.

Vorzugsweise unterscheiden sich die hinteren Seitenabschnitte von den vorderen Seitenabschnitten bezüglich mindestens einer, insbesondere mindestens zweier, weiter insbesondere mindestens dreier und weiter insbesondere mindestens vierer ihrer Primäreigenschaften ausgewählt aus der Gruppe Art des Materials, Flächengewicht, Atmungsaktivität, Dichte, Dehnbarkeit, Verschlusskraft, Flächenerstreckung, Dicke, Farbe. Art des Materials: Insbesondere, wenn beide Seitenabschnittskomponenten aus einem Vliesstoffmaterial gebildet sind, erweist es sich als vorteilhaft, wenn beispielsweise die vorderen Seitenabschnitte aus einem weicheren, hautfreundlicherem Vliesmaterial gebildet sind als die hinteren Seitenabschnitte, da die vorderen Seitenabschnitte beim Anlegen der Windel an den Körper bestimmungsgemäß innen zu liegen kommen. Weiterhin kann es vorteilhaft sein, die hinteren Seitenabschnitte aus einem zugfesteren Material zu bilden, da die Verschlussmittel vorzugsweise an den hinteren Seitenabschnitten angebracht sind und beim Anlegen der Windel starke Zugkräfte über die Verschlussmittel auf die Seitenabschnitte einwirken. Bevorzugte Differenzierungen hinsichtlich der Art des Materials lassen sich durch die verwendete Faserart, das Vliesbildungsverfahren oder Laminatbildungen realisieren.

Flächengewicht: Die zuvor genannten Anforderungen können vorzugsweise zumindest anteilig über eine Differenzierung des Flächengewichtes, gemessen in g/m², erreicht werden. Vorzugsweise unterscheidet sich das Flächengewicht der vorderen Seitenabschnitte um mindestens 10%, insbesondere um mindestens 20% und weiter insbesondere um mindestens 30% von dem der hinteren Seitenabschnitte. Das Flächengewicht der vorderen und/oder der hinteren Seitenabschnitte beträgt nach einem weiteren Erfindungsgedanken vorzugsweise 15-60 g/m², insbesondere 20-45 g/m², weiter insbesondere 25-40 g/m² und weiter insbesondere 28-35 g/m².

Atmungsaktivität: Vorzugsweise sind die vorderen und/oder die hinteren Seitenabschnitte durch einen luft- und/oder wasserdampfdurchlässigen Vliesstoff gebildet. Da das subjektive Empfinden des Tragekomforts von Zielgruppe zu Zielgruppe (beispielsweise bettlägerige Patienten vs. mobile Patienten) verschieden ist, kann es vorteilhaft sein, die Atmungsaktivität entweder der vorderen oder der hinteren Seitenabschnitte höher auszubilden. Vorzugsweise unterscheidet sich die Atmungsaktivität gemessen als Wasserdampfdurchlässigkeit (WVTR) nach DIN 53 122-1 (Ausgabe: 2001-08) der vorderen Seitenabschnitte von der der hinteren Seitenabschnitte um mindestens 5%, insbesondere um mindestens 10% und weiter insbesondere um mindestens 20%. Vorzugsweise beträgt dabei die Atmungsaktivität der vorderen und/oder hinteren Seitenabschnitte mindestens 1000 g/m²/24h, insbesondere mindestens 1500 g/m²/24h, weiter vorzugsweise mindestens 2000 g/m²/24h.

Dichte und Dicke: Die subjektiv empfundene Weichheit des Seitenabschnittsmaterials und damit eine wesentliche Komponente des Tragekomforts kann vorteilhaft auch über eine Differenzierung der Dichte und/oder Dicke des Materials gesteuert werden. Vorzugsweise unterscheidet sich die Dicke gemessen in mm, ermittelt bei einem Prüfdruck von 0,5 kPa, und/oder die Dichte gemessen in g/cm³, ermittelt aus den Größen Flächengewicht und Dicke des Materials, der vorderen Seitenabschnitte um mindestens 15%, insbesondere um mindestens 20% und weiter insbesondere um mindestens 25% von der Dichte und/oder der Dicke der hinteren Seitenabschnitte.

Dehnbarkeit: Unter Dehnung wird vorliegend das Verhältnis zwischen einer Längenzunahme eines Seitenabschnitts der Inkontinenzwegwerfwindel infolge einer Krafteinwirkung und der ursprünglichen Länge verstanden. In Gebrauch derartiger Inkontinenzwegwerfwindeln wirken auf die Seitenabschnitte insbesondere Kräfte in Umfangs- also Windelquerrichtung. Mit der Eigenschaft Dehnbarkeit ist somit das Ausmaß der Dehnung des Seitenabschnitts bei Einwirken einer Kraft in Windelquerrichtung gemeint. Das heißt, je höher das Ausmaß der Dehnung, desto höher ist die Dehnbarkeit. Vorzugsweise weist ein hinterer Seitenabschnitt bei in Gebrauch der Windel üblicher Krafteinwirkung eine größere Dehnbarkeit auf als ein vorderer Seitenabschnitt. Insbesondere weist nach der in DE102005048868A1 beschriebenen Prüfmethode ein hinterer Seitenabschnitt bei einer Krafteinwirkung von 45 N eine höhere Dehnung auf als ein vorderer Seitenabschnitt. Vorzugsweise weist ein hinterer Seitenabschnitt bei einer Krafteinwirkung von 45 N eine Dehnung von mindestens 20%, insbesondere mindestens 25% und weiter insbesondere mindestens 30% auf. Ein vorderer Seitenabschnitt hingegen weist bei einer Krafteinwirkung von 45 N lediglich eine Dehnung von vorzugsweise höchstens 15%, insbesondere höchstens von 10% und weiter insbesondere von höchstens 8% auf. Vorzugsweise ist zumindest ein hinterer Seitenabschnitt zumindest in Querrichtung elastisch dehnbar. Die Dehnbarkeit des Seitenabschnitts wird als elastisch bezeichnet, wenn bei kurzzeitiger Einwirkung einer Kraft eine Dehnung von mindestens 40% möglich ist und bei Wegnahme dieser Kraft eine Dehnung (bleibende Dehnung) von höchstens 20% verbleibt. In einer vorteilhaften Weiterbildung der Erfindung beträgt die elastische Dehnbarkeit eines hinteren Seitenabschnitts in Querrichtung mindestens 40%, insbesondere mindestens 50%. Nach einem weiteren Erfindungsgedanken beträgt das absolute Ausmaß der elastischen Dehnung eines hinteren Seitenabschnitts mindestens 3 cm, insbesondere mindestens 5 cm und weiter insbesondere mindestens 7 cm.

Verschlusskraft: Unter Verschlusskraft der Seitenabschnitte wird die Haltekraft zwischen den Verschlussmitteln der hinteren Seitenabschnitte und der Außenseite der Seitenabschnitte verstanden. Vorzugsweise sind die Haltekräfte zwischen den Verschlussmitteln und der Außenseite der hinteren Seitenabschnitte hierbei geringer als die Haltekräfte zwischen den Verschlussmitteln und der Außenseite der vorderen Seitenabschnitte. Dies führt in vorteilhafter Weise dazu, dass der Benutzer die Verschlussmittel bevorzugt an den vorderen Seitenabschnitten festlegt, was Passform und Tragekomfort der Windel deutlich zugute kommt. Die vorstehend oder nachfolgend erwähnten Haltekräfte werden vorzugsweise als Über-Bauch-Haltekräfte ermittelt. Die Über-Bauch-Haltekräfte sind nach der in EP1915977A1 beschriebenen Prüfmethode zu bestimmen. Die Haltekräfte als Über-Bauch-Haltekräfte ermittelt zwischen den insbesondere mechanischen Verschlusshilfen aufweisenden Verschlussmitteln und der Außenseite der vorderen Seitenabschnitte betragen vorzugsweise 58-90N/25mm, insbesondere 60-80N/25mm. Die Über-Bauch-Haltekräfte zwischen den insbesondere mechanische Verschlusshilfen aufweisenden Verschlussmitteln und der Außenseite der hinteren Seitenabschnitte sind vorzugsweise geringer als die Über-Bauch-Haltekräfte zwischen den Verschlussmitteln und der Außenseite der vorderen Seitenabschnitte, sie betragen vorzugsweise aber gleichwohl mindestens 15 N/25mm, insbesondere mindestens 30 N/25mm.

Vorzugsweise umfassen die mechanischen Verschlusshilfen an sich bekannte Kletthakenelemente. In einer weiteren Ausführungsform ist vorgesehen, dass zumindest ein Verschlussmittel, vorzugsweise alle Verschlussmittel außerdem eine klebende Verschlusshilfe, insbesondere eine Haftklebezone umfasst, beispielsweise derart, wie dies in EP1915977A1 offenbart ist.

Flächenerstreckung: In Weiterbildung der Erfindung erweist es sich als vorteilhaft, wenn die hinteren Seitenabschnitte eine größere, vorzugsweise eine um mindestens 10 %, insbesondere eine um mindestens 15% größere Flächenerstreckung aufweisen als die vorderen Seitenabschnitte. Insbesondere kann die Länge der hinteren Seitenabschnitte, also deren Erstreckung in Windellängsrichtung mindestens 10 cm, insbesondere mindestens 15 cm, weiter insbesondere mindestens 18 cm und weiter insbesondere mindestens 22 cm betragen. Es erweist sich weiterhin als vorteilhaft, wenn die Länge der hinteren Seitenabschnitte mindestens 10 %, insbesondere mindestens 15%, weiter insbesondere mindestens 20% und weiter insbesondere mindestens 22% der Gesamtlänge der Inkontinenzwegwerfwindel beträgt. Vorteilhafterweise beträgt die Gesamtlänge der Inkontinenzwegwerfwindel 50-120 cm, insbesondere 60-110 cm und weiter insbesondere 70-110 cm. Weiterhin erweist es sich als vorteilhaft, wenn die vorderen Seitenabschnitte eine geringere, insbesondere eine um mindestens 5%, weiter insbesondere eine um mindestens 10%, weiter insbesondere eine um mindestens 15% und weiter insbesondere eine um höchstens 50% geringere Längserstreckung aufweisen als die hinteren Seitenabschnitte. In Weiterbildung der Erfindung erweist es sich als vorteilhaft, wenn die Breite der Seitenabschnitte, also die Erstreckung der Seitenabschnitte über den Seitenrand des Windelhauptteils hinaus 10-40 cm, insbesondere 12-30 cm, weiter insbesondere 13-25 cm beträgt. Vorzugsweise weisen die vorderen Seitenabschnitte die gleiche Breite auf wie die hinteren Seitenabschnitte.

Farbe: Schließlich kann es vorteilhaft sein, die vorderen von den hinteren Seitenabschnitten hinsichtlich der Farbe zu differenzieren. Auch dies kann den Benutzern die Funktion der vorderen Seitenabschnitte als bevorzugte Landefläche der Verschlussmittel verdeutlichen.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:
- Figur 1: eine Draufsicht auf die körperabgewandte Seite einer erfindungsgemäß hergestellten Inkontinenzwegwerfwindel
- Figur 2: eine Teildraufsicht auf die körperabgewandte Seite der erfindungsgemäß hergestellten Inkontinenzwegwerfwindel nach Figur 1 mit noch gefalteten Seitenabschnitten
- Figur 3: eine schematische Darstellung eines erfindungsgemäßen Herstellungsverfahrens
- Figur 4: eine vergrößerte Teilansicht der in Figur 3 dargestellten Flachmaterialbahn nach Erzeugung der ersten Materialaussparung und vor dem Falten der Flachmaterialbahn
- Figur 5: eine weitere Variante des erfindungsgemäßen Herstellungsverfahrens

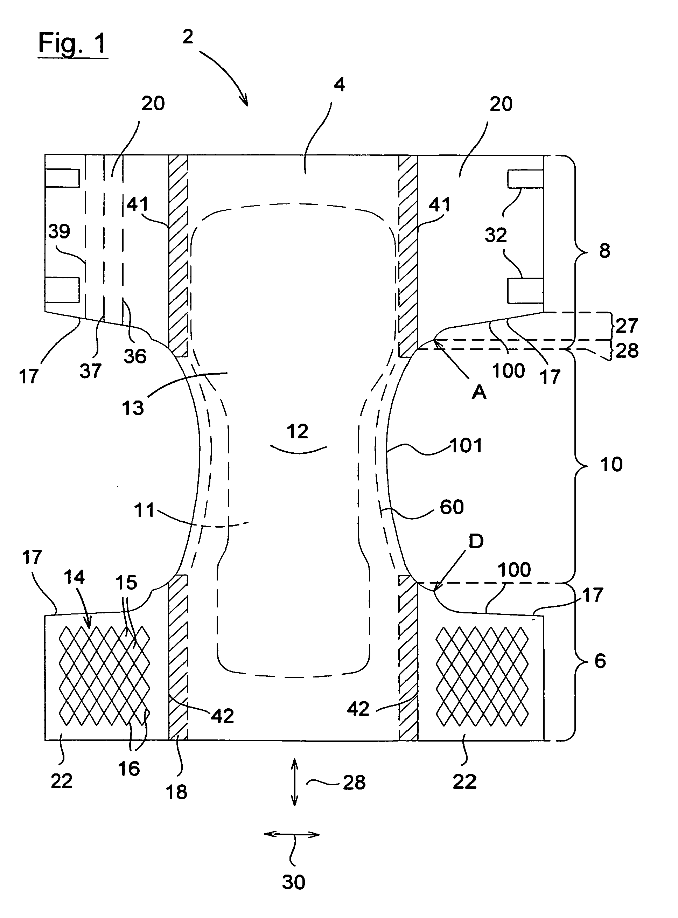

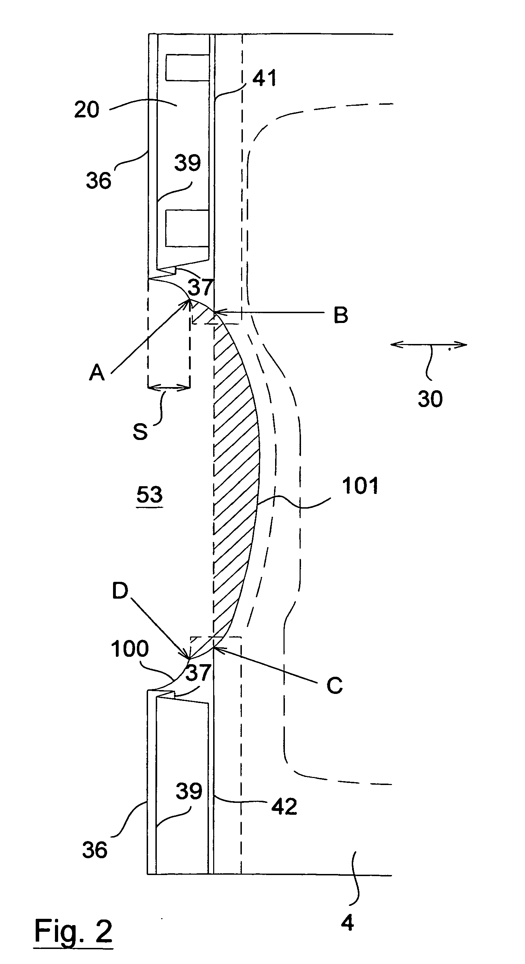

Figur 1 zeigt eine Draufsicht auf die Außenseite, also die körperabgewandte Seite einer auf erfindungsgemäße Weise herzustellenden Inkontinenzwegwerfwindel 2 des offenen Typs in ausgefalteter Konfiguration. Die Inkontinenzwegwerfwindel 2 umfasst einen Hauptteil 4 mit einem Vorderbereich 6, einem Rückenbereich 8 und einem in Längsrichtung dazwischen liegenden Schrittbereich 10. Außerdem angedeutet ist ein Saugkörper 12, der überlicherweise zwischen chassisbildenden Materialien des Hauptteils 4, also insbesondere zwischen einem flüssigkeitsdurchlässigen aus einem Vliesstoffmaterial gebildeten Topsheet 11 und einem im Wesentlichen flüssigkeitsundurchlässigen aus einem Folienmaterial gebildeten Backsheet 13 des Hauptteils 4 angeordnet ist. Das Backsheet 13 kann auch aus einem flüssigkeitsundurchlässigem Vliesstoff oder einem Vliesfolienlaminat gebildet sein, wobei die Vlieslage dann außen und die Folienlage innen zum Saugkörper gerichtet zu liegen kommt. Dies vermittelt der Inkontinenzwegwerfwindel 2 einen textilähnlichen Eindruck. Seitlich neben den Längsrändern des Saugkörpers 12 sind erste elastische Elemente 60 an den Hauptteil 4, zwischen Topsheet 11 und Backsheet 13 angefügt. Die elastischen Elemente 60 verlaufen im Wesentlichen in der Längsrichtung, also mit einer wesentlichen Komponente in Längsrichtung, wobei sie einen gekrümmten Verlauf entlang des dem Schrittbereich 10 zuzuordnenden Beinöffnungsbereichsabschnittes des Hauptteils 4 nehmen. Die Inkontinenzwegwerfwindel 2 umfasst des Weiteren vordere Seitenabschnitte 22 und hintere Seitenabschnitte 20, die als separate Komponenten, vorzugsweise auf Vliesstoffbasis, beidseits an den Hauptteil 4 angefügt sind. Die Seitenabschnitte 20, 22 sind in einem Überlappungsbereich 18 mit chassisbildenden Materialien des Hauptteils 4, also beispielsweise mit dem Backsheet 13 und/oder dem Topsheet 11 unlösbar verbunden. Die Seitenabschnitte 20, 22 erstrecken sich über die vorderen und hinteren Längsränder 42, 41 des Hauptteils in Querrichtung 30 hinaus. Unter vorderen und hinteren Längsrändern 42, 41 des Hauptteils werden im Rahmen der vorliegenden Erfindung diejenigen Längsrandbereiche des Hauptteils verstanden, an die die Seitenabschnitte angefügt sind und über welche diese sich hinaus erstrecken. Die Längserstreckung der vorderen und hinteren Seitenränder des Hauptteils 42, 41 definieren damit auch die Längserstreckung des Vorderbereiches 6 und des Rückenbereiches 8 der Inkontinenzwegwerfwindel 2, wie dies Figur 1 veranschaulicht. Die Position der ersten, zweiten und dritten Faltlinie 36, 37, 39 ist, um die Figur 1 nicht zu überfrachten, lediglich an einem hinteren linken Seitenabschnitt 20 angedeutet. Die noch näher zu betrachtende Figur 2 zeigt die linke Hälfte der Windel mit noch w-förmig gefalteten Seitenabschnitten 20, 22. Diese gefaltete Konfiguration ist dann in der gebrauchsfertigen Konfiguration um eine weitere Faltlinie, die ungefähr entlang des Längsrandes des Hauptteils 4 verläuft, nach innen auf die dem Körper zugewandte Seite des Hauptteils eingeschlagen (nicht dargestellt).

Die Seitenabschnitte 20, 22 sind dafür gedacht und bestimmt, im angelegten Zustand der Inkontinenzwegwerfwindel 2 miteinander verbunden zu werden, um einen in Umfangsrichtung durchgehenden Hüftbereich des Hygieneartikels zu bilden. Dabei werden jeweils die auf einer Seite des Hauptteils 4 vorgesehenen Seitenabschnitte 20, 22 miteinander verbunden. Hierzu sind an den hinteren Seitenabschnitten 20 vorzugsweise mechanische Verschlussmittel 32, insbesondere mit mechanischen Verschlusshilfen wie Kletthaken, vorgesehen, welche auf der Außenseite der vorderen und hinteren Seitenabschnitte 20, 22 lösbar festlegbar sind. Vorzugsweise sind die Verschlussmittel außerdem auf der Außenseite des Hauptteils lösbar festlegbar. Zumindest die Außenseite der vorderen Seitenabschnitte weist hierzu ein in Figur 1 nur schematisch angedeutetes Prägemuster 14 auf. Die durch Heißkalanderprägung erzeugten Fügebereiche sind durch eine Vielzahl von Linien gebildet, nämlich durch zwei Gruppen von innerhalb einer Gruppe jeweils parallel verlaufenden Linien, wobei sich die Linien der einen mit der der anderen Gruppe zur Ausbildung eines regelmäßigen Rautenmusters unter einem Winkel von 33 Grad schneiden, so dass inselartig angeordnete und verbundene, rautenförmige Schlaufenbereiche 15 von linienartigen Fügebereichen 16 umgeben sind. Die Fügebereiche 16 bildenden Linien haben im dargestellten Fall eine Breite von 1,0 mm und eine Prägetiefe von 0,6 mm. Der Abstand zweier benachbarter parallel verlaufender Linien beider Gruppen von Linien beträgt 4,7 mm. Die Prägefläche, also die Summe der Fläche aller Fügebereiche 16 bezogen auf die Gesamtfläche des Prägemusters (Fügebereiche + Schlaufenbereiche), beträgt 32 %. Die Verschlussmittel 32 der hinteren Seitenabschnitte 20 sind mit diesen Schlaufenbereichen 15 sicher in Eingriff bringbar. Die Über-Bauch-Haltekräfte zwischen den Verschlussmitteln 32 und der Außenseite der vorderen Seitenabschnitte 22 betragen vorzugsweise mindestens 58 N/25mm. Sowohl die vorderen Seitenabschnitte 22 als auch die hinteren Seitenabschnitte 20 sind aus einem Vliesstoffmaterial, im dargestellten Fall aus einem PP-Spinnvlies, gebildet. Das Flächengewicht des Vliesstoffmaterials der vorderen Seitenabschnitte beträgt 30 g/m². Die Faserstärke der das Vliesstoffmaterial bildenden Fasern beträgt 2 dtex.

Wie aus Figur 1 erkennbar ist, weisen die hinteren Seitenabschnitte 20 zudem eine größere Flächenerstreckung auf als die vorderen Seitenabschnitte 22.

Vordere und hintere Seitenabschnitte 20, 22 weisen an ihren schrittzugewandten Rändern 17 konturierte Beinausschnittbereiche auf. Die konturierten Beinausschnittbereiche der Seitenabschnitte 20, 22 weisen ihrerseits einen äußeren Teilabschnitt 27 und einen inneren Teilabschnitt 28 auf. Der äußere Teilabschnitt 27 erstreckt sich von einem jeweiligen äußeren Längsrand der Seitenabschnitte bis zu einem Punkt A (im Falle der hinteren Seitenabschnitte 20) bzw. bis zu einem Punkt D (im Falle der vorderen Seitenabschnitte 22). Der sich an diese Punkte A und D jeweils in Richtung Hauptteil 4 anschließende Bereich bildet den jeweiligen inneren Teilabschnitt 28. Dieser geht dann stetig über in den jeweiligen Beinausschnittbereich des Hauptteils 4. Die Punkte A und D stellen gewissermaßen jeweils einen Punkt geringfügiger Unstetigkeit der ansonsten stetigen, insbesondere kurvenförmigen Konturierung der Beinausschnittbereiche dar. Herbeigeführt ist diese Ausgestaltung der Beinausschnittbereiche der Seitenabschnitte durch das unten näher beschriebene erfindungsgemäße Herstellungsverfahren, welches die Bildung erster und zweiter Materialaussparungen entlang erster 100 und zweiter Schnittlinien 101 umfasst.

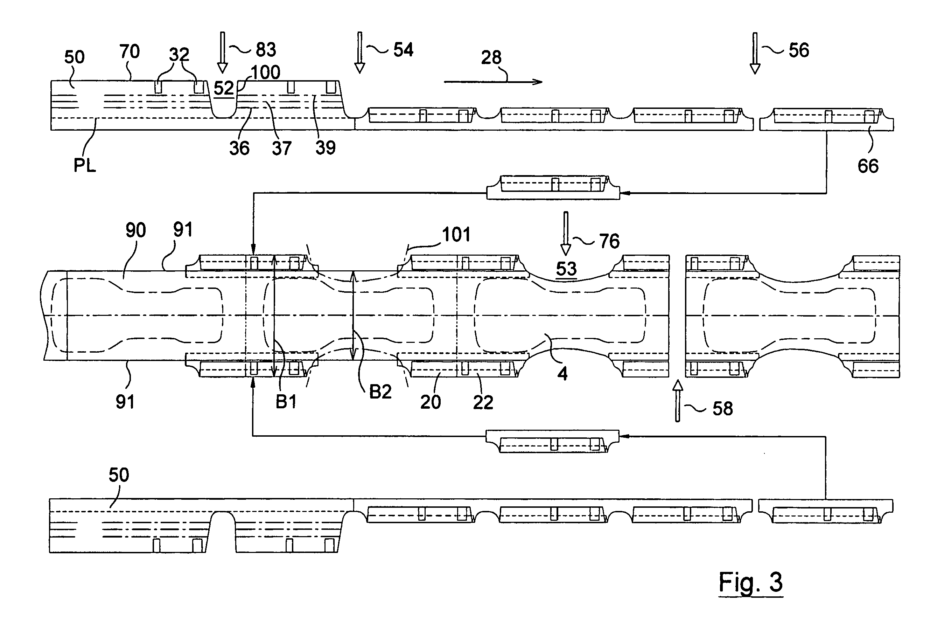

Für die Herstellung dieser Windel 2 erweist es sich als vorteilhaft, wenn eine die Seitenabschnitte 20, 22 bildende Materialbahn in Längsrichtung endlos zugeführt wird. Ein hierfür anwendbares erfindungsgemäßes Verfahren wird nun anhand der Figur 3 beschrieben:

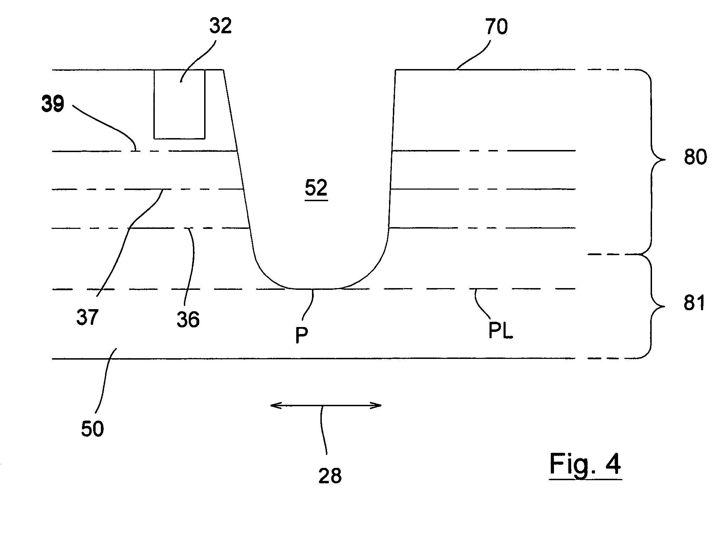

Figur 3 zeigt schematisch das Zuführen und Konfigurieren von zwei Flachmaterialbahnen 50, aus denen die linken und rechten, vorderen und hinteren Seitenabschnitte 20, 22 der Windel 2 gebildet werden. Die Flachmaterialbahnen 50 werden in der Längsrichtung 28 (Längsfertigung) gefördert. Zur Bildung eines Teilabschnitts der späteren Konturierung der Seitenabschnitte 20, 22 auf ihrer dem Schrittbereich 10 zugewandten Seite werden an einem jeweiligen Seitenrand 70 der Flachmaterialbahnen 50 mittels eines Trennwerkzeuges, vorzugsweise einer rotierenden Messerwalze, an einer ersten Schnittstation 83 entlang einer ersten Schnittlinie 100 die ersten Materialaussparungen 52 gebildet. Eine vergrößerte Teilansicht einer mit der Materialaussparung 52 versehenen Flachmaterialbahn 50 zeigt Figur 4. Erkennbar ist, dass eine gedachte Parallele PL zur Längsrichtung 28, welche an den Punkt P der maximalen Erstreckung des Ausschnittes quer zur Längsrichtung 28 angelegt ist, einen äußeren Teilbereich 80, in dem die Materialaussparungen 52 angeordnet sind, und einen inneren Teilbereich 81 der Bahn definiert.

Vor oder nach dem Ausbilden der Materialaussparungen 52 werden Verschlussmittel 32 in der Fertigungsrichtung zwischen zwei aufeinander folgenden Öffnungen 52 appliziert. Es handelt sich hierbei um an sich bekannte klebend und/oder mechanisch haftende Verschlussmittel 32, beispielsweise in Form von streifenförmigen Verschlusstapes.

Die Flachmaterialbahn 50 wird in einer Faltstation 54 von außen um drei in der Längsrichtung 28 verlaufende Faltlinien 36, 37, 39 auf sich selbst gefaltet, so dass sich eine w-förmige Faltung ergibt. Dabei ist erfindungsgemäß vorgesehen, dass die erste Faltlinie 36, also die am weitesten innen liegende Faltlinie innerhalb eines jeweiligen äußeren Teilbereiches 80 verläuft. Die zweite Faltlinie 37 ist näher zum Seitenrand 70 angeordnet ist als die erste Faltlinie 36 und die dritte Faltlinie 39 ist näher zum Seitenrand 70 angeordnet ist als die zweite Faltlinie 37. Damit verlaufen auch die weiteren Faltlinien 37, 39 innerhalb des äußeren Teilbereichs 80. Erforderlichenfalls werden die aufeinander gefalteten Teilabschnitte der Flachmaterialbahn lösbar aneinander fixiert.

In einer jeweiligen Vereinzelungsstation 56 werden die in Längsrichtung noch kontinuierlichen Flachmaterialbahnen 50 quer zur Längsrichtung 28 in Materialabschnitte 66 getrennt. Die Trennung erfolgt vorzugsweise im Bereich der maximalen Erstreckung des die erste Materialaussparung bildenden Ausschnittes quer zur Längsrichtung. Diese Materialabschnitte 66 werden dann auf die ebenfalls in der Längsrichtung geförderte Hauptteilbahn 90 appliziert und dort in wie oben beschrieben gefaltetem Zustand an Längsrandabschnitten des späteren Windelhauptteils 4 unlösbar zur Bildung von Seitenabschnitten 20, 22 befestigt. Die noch endlose Windelbahn weist nun im Bereich der gefalteten Seitenabschnitte eine maximale Breite B1 (Erstreckung quer zur Längsrichtung 28) von 510 mm auf; im Schrittbereich beträgt die minimale Breite B2 330 mm. Das Verhältnis der Breiten beträgt also im dargestellten Fall 1,55 und ist damit klein genug, um die noch unfertige Windelbahn mit anhaltend hoher Geschwindigkeit sicher und präzise weiter zu fördern und weiter maschinell zu bearbeiten.

Das Abtrennen der Materialabschnitte 66, das Zuführen zu der Hauptteilbahn 90 und das nachfolgende Anfügen der Materialabschnitte 66 an beide Seitenränder der Hauptteilbahn 90 erfolgt bevorzugt mittels dem Fachmann an sich bekannter in Figur 3 nicht dargestellter Slip-Cut- oder auch Cut-and-place-Units genannter Vorrichtungen.

Bei den in Figur 3 schematisch dargestellten Materialabschnitten 66 nach der Abtrennung von den endlosen Flachmaterialbahnen 50 kann es sich um einen einer einzigen Windel zuzuordnenden Seitenabschnitt handeln. Vorteilhaft ist allerdings wie in Figur 3 dargestellt, dass dieser Materialabschnitt Seitenabschnitte zweier in der Längsrichtung 28 bzw. Produktionsrichtung aufeinander folgend geförderter Windeln bildet. Die Seitenabschnitte werden im letzteren Fall dann vorzugsweise und zweckmäßigerweise mit der schlussendlichen Vereinzelung der Windeln von einer endlosen Bahn wie weiter unten beschrieben ebenfalls quer zur Längsrichtung an einer Vereinzelungsstation abgetrennt. Man erkennt, dass Materialabschnitte 66 zur Bildung von Seitenabschnitten 20, 22 an eine endlose Bahn 90, welche die Windelhauptteile 4 bildet, angefügt werden. Jeder Materialabschnitt 66 umfasst jeweils die Seitenabschnitte 20, 22 zweier in der Längsrichtung 28 bzw. Produktionsrichtung aufeinander folgend geförderter Windeln. Bei dem Prozess gemäß Figur 3 werden die Windeln derart gefertigt, dass bei in der Längsrichtung 28 aufeinander folgend geförderten Windeln der Rückenbereich 8 der einen Windel an den Vorderbereich 6 der anderen Windel anschließt.

Es wäre auch denkbar und vorteilhaft, dass in einen Herstellungsprozess einer Windel bis zu vier der in Figur 3 dargestellten Flachmaterialbahnen implementiert werden, wobei dann je eine Flachmaterialbahn hintere oder vordere linke oder hintere oder vordere rechte Seitenabschnitte bereitstellen kann. Auf diese Weise können dann auch verschiedene Bahnmaterialien zur Bildung der Seitenabschnitte im Vorderbereich und im Rückenbereich eingesetzt werden.

In einem nachfolgenden ebenfalls in Figur 3 dargestellten Verfahrensschritt werden an einer weiteren jeweiligen zweiten Schnittstation 76 die zweiten Materialaussparungen 53 entlang einer zweiten Schnittlinie 101 gebildet, wobei die zweite Schnittlinie 101 die Seitenabschnitte 20, 22 und einen jeweiligen Längsrand 91 der Windelhauptteilbahn 90 erfasst, und wobei die zweite Schnittlinie 101 die erste Schnittlinie 100 kreuzt, derart dass sich die zweite Schnittlinie nicht durch die erste Faltlinie der Seitenabschnitte 20, 22 erstreckt.

Durch diese zweite Materialaussparung 53 erhält die Beinöffnungskontur seine endgültige Form. In Figur 3 ist der Verlauf der in diesem Verfahrensschritt erfolgenden zweiten Materialaussparung schematisch als gestrichelte zweite Schnittlinie 101 an der Windel unmittelbar vor der Schnittstation 76 angedeutet. Im Detail ist der Verlauf dieser Schnittlinie 101 außerdem in Figur 2 zu erkennen: Die zweiten Materialaussparungen 53 werden entlang einer zweiten Schnittlinie 101 gebildet, die sich ausgehend von einem Punkt A auf der ersten Schnittlinie 100 eines hinteren Seitenabschnitts 20, das heißt von dem Punkt, in dem sich die Schnittlinien 100, 101 erstmals kreuzen, in einer Kurve nach innen in Richtung Schrittbereich 10 zunächst bis zu einem Punkt B eines hinteren Seitenrandes 41 des Hauptteils 4 und dann in den Hauptteil 4 hinein erstreckt. Die zweite Schnittlinie 101 erstreckt sich dann weiter durch den Schrittbereich 10 am Längsrand des Hauptteils 4 entlang, vorzugsweise ohne die elastischen Elemente 60 und den Saugkörper 12 zu erfassen, und daran anschließend in einer Kurve nach außen durch einen Punkt C des vorderen Seitenrandes 42 des Hauptteils 4 und schließlich bis zu einem Punkt D auf der ersten Schnittlinie 100 des vorderen Seitenabschnitts 22, das heißt bis zu dem Punkt, in dem sich die Schnittlinien 100, 101 erneut kreuzen. Der beschriebene Verlauf der Schnittlinie 101 muss nicht bedeuten, dass der Verfahrensschritt des Herausschneidens tatsächlich am Punkt A beginnen muss. Der Schnittvorgang selbst kann natürlich auch in Punkt D beginnen und sich dann über Punkt C, und B nach Punkt A fortsetzen. Der Schnitt könnte auch an einem anderen beliebigen Punkt auf der beschriebenen Schnittlinie 101 ansetzen und sich dann entlang der Schnittlinie in beide Richtungen fortsetzen. Im Ergebnis resultiert jedenfalls der Verlauf der mit Bezug zu Figur 2 beschriebenen Schnittlinie 101. Punkt A und Punkt D sind in Querrichtung 30 nach innen, also in Richtung auf den Hauptteil 4 der Windel 2 von der jeweiligen ersten Faltlinie 36 um den Abstand S beabstandet, und zwar um 4-10 mm, so dass die zweite Schnittlinie 101 die jeweilige erste Faltlinie 36 der Seitenabschnitte 20, 22 nicht erfasst. Auch die weiteren Faltlinien 37, 39 des Seitenabschnitts 20 werden durch die zweite Schnittlinie 101 nicht erfasst. Die herausgeschnittenen Materialkomponenten der Seitenabschnitte 20, 22 und des Hauptteils 4 sind in Figur 2 schraffiert dargestellt. Es resultiert die mit Bezug zu Figur 1 beschriebene Kontur der Beinausschnittbereiche mit inneren und äußeren Teilabschnitten 27, 28 im Bereich der Seitenabschnitte 20, 22 und einem dazwischen liegenden Beinausschnittbereich des Hauptteils 4.

Schließlich erfolgt an einer Windelvereinzelungsstation 58 (Figur 3) die Vereinzelung der zuvor noch endlosen Windelbahn quer zur Längsrichtung, wobei die Seitenabschnitte erst gleichzeitig mit dem Vereinzelungsschnitt in vordere und hintere Seitenabschnitte 20, 22 getrennt werden.

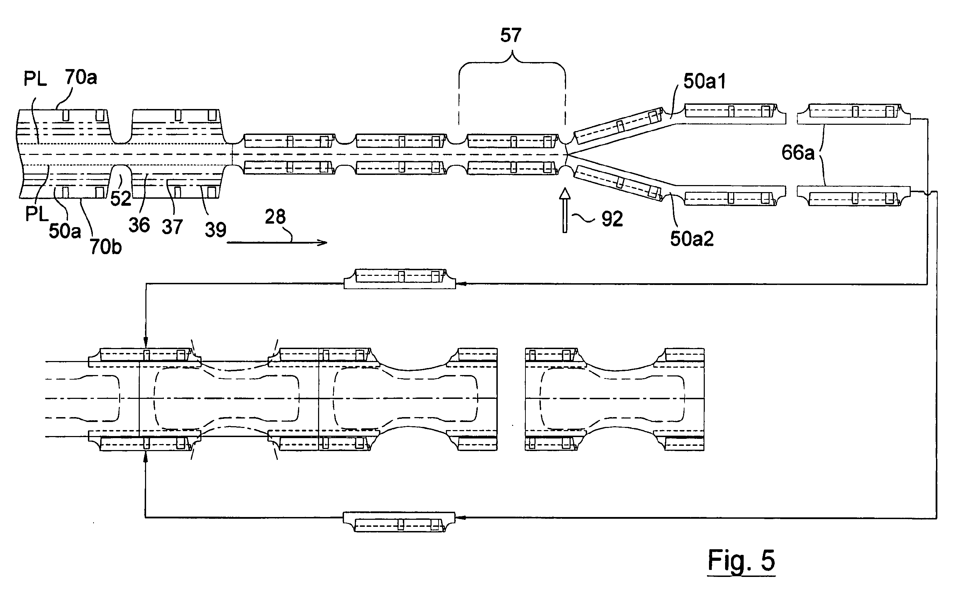

Wie in Figur 5 dargestellt ist denkbar und vorteilhaft, dass eine Flachmaterialbahn 50a doppelnutzig ausgeführt ist, derart dass aus einem Längsabschnitt 57 der Flachmaterialbahn 50a zwei Materialabschnitte 66a hervorgehen, wobei die ersten Materialaussparungen 52 und das Falten der Flachmaterialbahn beidseits, also an einem ersten 70a und an einem zweiten Längsrand 70b erfolgt und die Flachmaterialbahn 50a in Längsrichtung an einer Trennstation 92 geteilt wird, noch bevor von den dadurch erhaltenen Teilbahnen 50a1, 50a2 Materialabschnitte 66a abgetrennt werden. Solchenfalls könnte wie in Figur 5 dargestellt die eine Teilbahn zur Bildung von rechten Seitenabschnitten und die andere Teilbahn zur Bildung von linken Seitenabschnitten dienen. Die Flachmaterialbahn kann hierbei wie dargestellt nach der Bildung der ersten Materialaussparungen und nach dem Falten der Flachmaterialbahn in Längsrichtung geteilt werden oder bereits in einem vorgelagerten Verfahrensschritt. Insoweit in Figur 5 gleiche Bezugszeichen wie in Figuren 3 und 4 verwendet wurden, kennzeichnen diese analoge Komponenten.

Durch die erfindungsgemäße Verfahrensführung wird erreicht, dass die noch unfertige Windelbahn mit bereits angefügten Seitenabschnitten in der schnelllaufenden Windelmaschine sicher und stabil geführt und bearbeitet werden kann. Hierin liegt eine wesentliche Verbesserung des Herstellverfahrens.

## Patentansprüche

1. Verfahren zum Herstellen einer Wegwerfwindel des offenen Typs mit einem Hauptteil (4), umfassend einen Vorderbereich (6) mit vorderen seitlichen Längsrändern (42), einen Rückenbereich (8) mit hinteren seitlichen Längsrändern (41) und einem in Längsrichtung (28) dazwischen liegenden zwischen den Beinen eines Benutzers zu liegen kommenden Schrittbereich (10), wobei der Hauptteil (4) einen Saugkörper (12) umfasst, und mit beidseits an den Rückenbereich (8) angefügten hinteren Seitenabschnitten (20) und beidseits an den Vorderbereich (6) angefügten vorderen Seitenabschnitten (22), welche sich in Querrichtung (30) über die seitlichen vorderen und hinteren Längsränder (42, 41) des Hauptteils (4) hinaus erstrecken und den Vorderbereich (6) und den Rückenbereich (8) im angelegten Zustand der Inkontinenzwegwerfwindel miteinander verbinden, wobei zur Bildung von Beinausschnittbereichen die Seitenabschnitte (20, 22) zumindest an den dem Schrittbereich (10) zugewandten Rändern (17) schräg zur Längsrichtung (28) verlaufend oder kurvenförmig ausgebildet sind und der Hauptteil (4) zumindest im Schrittbereich sanduhrförmig ausgebildet ist, umfassend die folgenden Verfahrensschritte:
Zuführen einer endlosen Flachmaterialbahn (50, 50a) in einer Längsrichtung (28) zur Herstellung von Materialabschnitten (66, 66a) für die Bildung der Seitenabschnitte (20, 22),
Bildung von ersten Materialaussparungen (52) entlang einer ersten Schnittlinie (100) an einem Seitenrand (70, 70a, 70b) der Flachmaterialbahn (50, 50a) zur Erzeugung von konturierten Ausschnitten in der Flachmaterialbahn (50, 50a), wobei eine gedachte Parallele PL zur Längsrichtung, welche an den Punkt P der maximalen Erstreckung des Ausschnittes quer zur Längsrichtung (28) angelegt ist, einen äußeren Teilbereich (80) und einen inneren Teilbereich (81) der Flachmaterialbahn (50) definiert
Falten der Flachmaterialbahn (50, 50a) um mindestens eine in Längsrichtung (28) verlaufende erste Faltlinie (36), wobei die Faltlinie (36) innerhalb des äußeren Teilbereiches (80) verläuft,
Abtrennen von Längsabschnitten der gefalteten Flachmaterialbahn (50, 50a) zur jeweiligen Bildung von Materialabschnitten (66, 66a)
unlösbares Fixieren der Materialabschnitte (66, 66a) an einem jeweiligen Längsrand (91) einer Windelhauptteilbahn (90) zur Bildung von Seitenabschnitten (20, 22)
Bildung von zweiten Materialaussparungen (53) entlang einer zweiten Schnittlinie (101), wobei die zweite Schnittlinie (101) die Seitenabschnitte (20, 22) und einen jeweiligen Längsrand (91) der Windelhauptteilbahn (90) erfasst, und wobei die zweite Schnittlinie (101) die erste Schnittlinie (100) kreuzt, derart dass sich die zweite Schnittlinie (101) nicht durch die erste Faltlinie (36) der vorderen und hinteren Seitenabschnitte (20, 22) erstreckt.

2. Verfahren nach Anspruch 1 **dadurch gekennzeichnet, dass** sich die zweite Schnittlinie (101) ausgehend von einem Punkt A auf der ersten Schnittlinie (100) eines hinteren Seitenabschnittes (20) in einer Kurve nach innen in Richtung Schrittbereich (10) zunächst bis zu einem Punkt B eines jeweiligen hinteren Seitenrandes (41) des Hauptteils (4) und dann in den Hauptteil (4) hinein erstreckt und wobei der Punkt A in Querrichtung nach innen von der ersten Faltlinie (36) beabstandet ist und die Schnittlinie (101) sich weiter durch den Schrittbereich (10) des Hauptteils (4) und daran anschließend in einer Kurve nach außen durch einen Punkt C des vorderen Seitenrandes (42) des Hauptteils (4) und schließlich bis zu einem Punkt D auf der ersten Schnittlinie (100) der vorderen Seitenabschnitte (22) erstreckt, wobei der Punkt D in Querrichtung nach innen von der ersten Faltlinie (36) der vorderen Seitenabschnitte (22) beabstandet ist.

3. Verfahren nach Anspruch 2 **dadurch gekennzeichnet, dass** der Punkt A um 2-60 mm, nach innen von der ersten Faltlinie (36) beabstandet ist.

4. Verfahren nach Anspruch 3 **dadurch gekennzeichnet, dass** der Punkt D um 2-60 mm, nach innen von der ersten Faltlinie (36) beabstandet ist.

5. Verfahren nach einem der vorangegangenen Ansprüche, **dadurch gekennzeichnet, dass** die Flachmaterialbahn (50) um eine zweite in Längsrichtung (28) verlaufende Faltlinie (37 auf sich selbst gefaltet ist, wobei die zweite Faltlinie (37) näher zum Seitenrand (70, 70a, 70b) angeordnet ist als die erste Faltlinie (36).

6. Verfahren nach einem der vorangegangenen Ansprüche, **dadurch gekennzeichnet, dass** die Flachmaterialbahn (50, 50a) um eine dritte in Längsrichtung (28) verlaufende Faltlinie (39) auf sich selbst gefaltet ist, wobei die dritte Faltlinie (39) näher zum Seitenrand (70, 70a, 70b) angeordnet ist als die zweite Faltlinie (37).

7. Verfahren nach einem der vorstehenden Ansprüche, **dadurch gekennzeichnet, dass** die aufeinander gefalteten Teilabschnitte der Flachmaterialbahn (50, 50a) lösbar fixiert werden.

8. Verfahren nach einem der vorstehenden Ansprüche **dadurch gekennzeichnet, dass** die Flachmaterialbahn (50a) doppelnutzig ausgeführt ist, derart dass aus einem Längsabschnitt (57) der Flachmaterialbahn (50a) zwei Materialabschnitte (66a) hervorgehen, wobei die ersten Materialaussparungen (52) und das Falten der Flachmaterialbahn (50a) beidseits, also an einem ersten (70a) und an einem zweiten Längsrand (70b) erfolgt und die Flachmaterialbahn (50a) in Längsrichtung geteilt wird.

9. Verfahren nach Anspruch 8 **dadurch gekennzeichnet, dass** die Flachmaterialbahn (50a) nach der Bildung der ersten Materialaussparungen (52) und nach dem Falten der Flachmaterialbahn (50a) in Längsrichtung (28) geteilt wird.

10. Verfahren nach einem der vorstehenden Ansprüche **dadurch gekennzeichnet, dass** Verschlussmittel (32) auf die Flachmaterialbahn (50, 50a) aufgebracht werden.

11. Verfahren nach einem der vorstehenden Ansprüche **dadurch gekennzeichnet, dass** die Wegwerfwindeln (2) derart gefertigt werden, dass bei in der Längsrichtung (28) aufeinander folgend geförderten Windeln der Rückenbereich (8) der einen Windel an den Rückenbereich (8) der anderen Windel anschließt und der Vorderbereich (6) der einen Windel an den Vorderbereich (6) der anderen Windel anschließt.

12. Verfahren nach einem der vorstehenden Ansprüche **dadurch gekennzeichnet, dass** die Wegwerfwindeln (2) derart gefertigt werden, dass bei in der Längsrichtung (28) aufeinander folgend geförderten Windeln der Rückenbereich (8) der einen Windel an den Vorderbereich (6) der anderen Windel anschließt und der Vorderbereich (6) der einen Windel an den Rückenbereich (8) der anderen Windel anschließt.

13. Verfahren nach einem der vorstehenden Ansprüche **dadurch gekennzeichnet, dass** je ein quer zur Längsrichtung (28) abgetrennter Materialabschnitt (66, 66a) Seitenabschnitte (20, 22) von zwei aufeinander folgend geförderten Windeln bildet.

14. Verfahren nach einem der vorstehenden Ansprüche **dadurch gekennzeichnet, dass** die Seitenabschnitte (20, 22) von Vorderbereich (6) und Rückenbereich (8) verschieden ausgebildet sind.

15. Verfahren nach einem der vorstehenden Ansprüche **dadurch gekennzeichnet, dass** nach dem Verfahrensschritt des unlösbares Fixierens der Materialabschnitte (66, 66a) an einem jeweiligen Längsrand (91) einer Windelhauptteilbahn (90) das Verhältnis der maximalen Breite B1 der in der Längsrichtung geförderten Windelbahn zur minimalen Breite B2 der Windelbahn im Schrittbereich vor der Bildung der zweiten Materialaussparungen mindestens 1,1 und höchstens 2 beträgt.

## Claims

1. Process for producing a disposable diaper of the open type with a main part (4), comprising a front region (6) with front lateral longitudinal edges (42), a rear region (8) with rear lateral longitudinal edges (41) and a crotch region (10), lying in between in the longitudinal direction (28) and coming to lie between the legs of a user, the main part (4) comprising an absorbent pad (12), and with rear side portions (20), joined to the rear region (8) on both sides, and front side portions (22), joined to the front region (6) on both sides, extending in the transverse direction (30) beyond the lateral front and rear longitudinal edges (42, 41) of the main part (4) and connecting the front region (6) and the rear region (8) to one another in the put-on state of the disposable incontinence diaper, wherein the side portions (20, 22) are formed as running obliquely in relation to the longitudinal direction (28), or in a curve-shaped manner, at least at the edges (17) facing the crotch region (10), in order to form leg cutout regions, and the main part (4) is formed in an hourglass-shaped manner, at least in the crotch region, comprising the following process steps:
supplying a continuous web of flat material (50, 50a) in a longitudinal direction (28) in order to produce portions of material (66, 66a) for the forming of the side portions (20, 22),
forming first material clearances (52) along a first cutting line (100) at a side edge (70, 70a, 70b) of the web of flat material (50, 50a) in order to produce contoured cutouts in the web of flat material (50, 50a), an imaginary parallel line PL, which is parallel to the longitudinal direction and placed at the point P of maximum extent of the cutout transversely in relation to the longitudinal direction (28), defining an outer sub-region (80) and an inner sub-region (81) of the web of flat material (50),
folding the web of flat material (50, 50a) about at least one first folding line (36) running in the longitudinal direction (28), the folding line (36) running within the outer sub-region (80),
severing longitudinal portions of the folded web of flat material (50, 50a) for the respective forming of portions of material (66, 66a),
undetachably fixing the portions of material (66, 66a) to a respective longitudinal edge (91) of a web for the main part (90) of the diaper for the forming of side portions (20, 22),
forming second material clearances (53) along a second cutting line (101), the second cutting line (101) taking in the side portions (20, 22) and a respective longitudinal edge (91) of the web for the main part (90) of the diaper, and the second cutting line (101) crossing the first cutting line (100) in such a way that the second cutting line (101) does not extend through the first folding line (36) of the front and rear side portions (20, 22).

2. Process according to Claim 1, **characterized in that** the second cutting line (101) extends from a point A on the first cutting line (100) of a rear side portion (20) in a curve inwardly in the direction of the crotch region (10), initially as far as a point B of a respective rear side edge (41) of the main part (4) and then into the main part (4), the point A being at a distance from the first folding line (36) inwardly in the transverse direction, and the cutting line (101) extends further through the crotch region (10) of the main part (4) and, following that, in a curve outwardly through a point C of the front side edge (42) of the main part (4), finally as far as a point D on the first cutting line (100) of the front side portions (22), the point D being at a distance from the first folding line (36) of the front side portions (22) inwardly in the transverse direction.

3. Process according to Claim 2, **characterized in that** the point A is at a distance from the first folding line (36) inwardly of 2-60 mm.

4. Process according to Claim 3, **characterized in that** the point D is at a distance from the first folding line (36) inwardly of 2-60 mm.

5. Process according to one of the preceding claims, **characterized in that** the web of flat material (50) is folded on itself about a second folding line (37), running in the longitudinal direction (28), the second folding line (37) being arranged closer to the side edge (70, 70a, 70b) than the first folding line (36).

6. Process according to one of the preceding claims, **characterized in that** the web of flat material (50, 50a) is folded on itself about a third folding line (39), running in the longitudinal direction (28), the third folding line (39) being arranged closer to the side edge (70, 70a, 70b) than the second folding line (37).

7. Process according to one of the preceding claims, **characterized in that** the sub-portions of the web of flat material (50, 50a) that are folded on one another are detachably fixed.

8. Process according to one of the preceding claims, **characterized in that** the web of flat material (50a) is made to produce double blanks, in such a way that two portions of material (66a) are obtained from one longitudinal portion (57) of the web of flat material (50a), the first material clearances (52) and the folding of the web of flat material (50a) taking place on both sides, that is to say at a first (70a) and a second longitudinal edge (70b), and the web of flat material (50a) being divided in the longitudinal direction.

9. Process according to Claim 8, **characterized in that** the web of flat material (50a) is divided in the longitudinal direction (28) after the forming of the first material clearances (52) and after the folding of the web of flat material (50a).

10. Process according to one of the preceding claims, **characterized in that** closure means (32) are applied to the web of flat material (50, 50a).

11. Process according to one of the preceding claims, **characterized in that** the disposable diapers (2) are produced in such a way that, with diapers conveyed following one another in the longitudinal direction (28), the rear region (8) of the one diaper follows on from the rear region (8) of the other diaper and the front region (6) of the one diaper follows on from the front region (6) of the other diaper.

12. Process according to one of the preceding claims, **characterized in that** the disposable diapers (2) are produced in such a way that, with diapers conveyed following one another in the longitudinal direction (28), the rear region (8) of the one diaper follows on from the front region (6) of the other diaper and the front region (6) of the one diaper follows on from the rear region (8) of the other diaper.

13. Process according to one of the preceding claims, **characterized in that** a portion of material (66, 66a) severed transversely in relation to the longitudinal direction (28) forms side portions (20, 22) of two diapers conveyed following one another.

14. Process according to one of the preceding claims, **characterized in that** the side portions (20, 22) of the front region (6) and the rear region (8) are formed differently.

15. Process according to one of the preceding claims, **characterized in that**, after the process step of undetachably fixing the portions of material (66, 66a) to a respective longitudinal edge (91) of a web for the main part (90) of the diaper, the ratio of the maximum width B1 of the diaper web conveyed in the longitudinal direction to the minimum width B2 of the diaper web in the crotch region before the forming of the second material clearances is at least 1.1 and at most 2.

## Revendications

1. Procédé de production d'une couche jetable du type ouvert avec une partie principale (4), comprenant une région antérieure (6) avec des bords longitudinaux latéraux antérieurs (42), une région dorsale (8) avec des bords longitudinaux latéraux postérieurs (41) et une région d'entrejambe (10) située entre celles-ci dans la direction longitudinale (28) et venant se placer entre les jambes d'un utilisateur, dans lequel la partie principale (4) comprend un corps absorbant (12), et avec des parties latérales postérieures (20) assemblées de part et d'autre à la région dorsale (8) et des parties latérales antérieures (22) assemblées de part et d'autre à la région antérieure (6), qui s'étendent en direction transversale (30) au-delà des bords longitudinaux latéraux antérieurs et postérieurs (42, 41) de la partie principale (4) et qui assemblent l'une à l'autre la région antérieure (6) et la région dorsale (8) dans l'état appliqué de la couche d'incontinence jetable, dans lequel, pour la formation des régions de découpe des jambes, les parties latérales (20, 22) sont, au moins sur les bords (17) tournés vers la région d'entrejambe (10), configurées en oblique par rapport à la direction longitudinale (28) ou sous forme courbe, et la partie principale (4) est réalisée en forme de sablier au moins dans la région d'entrejambe, comprenant les étapes suivantes:
- amener une bande sans fin de matériau plat (50, 50a) dans une direction longitudinale (28) pour la production de parties de matériau (66, 66a) destinées à la formation des parties latérales (20, 22);
- former des premières découpes de matériau (52) le long d'une première ligne de coupe (100) sur un bord latéral (70, 70a, 70b) de la bande de matériau plat (50, 50a) pour produire des découpes conformées dans la bande de matériau plat (50, 50a), dans lequel une parallèle imaginaire PL à la direction longitudinale, qui est menée au point P de l'extension maximale de la découpe transversalement à la direction longitudinale (28), définit une région partielle extérieure (80) et une région partielle intérieure (81) de la bande de matériau plat (50);
- plier la bande de matériau plat (50, 50a) autour d'au moins une première ligne de pliage (36) s'étendant dans la direction longitudinale (28), dans lequel la ligne de pliage (36) s'étend à l'intérieur de la région partielle extérieure (80);
- séparer des parties longitudinales de la bande de matériau plat pliée (50, 50a) pour former respectivement des parties de matériau (66, 66a);
- fixer de manière inséparable les parties de matériau (66, 66a) à un bord longitudinal respectif (91) d'une bande de partie principale de couche (90) pour la formation de parties latérales (20, 22);
- former des deuxièmes découpes de matériau (53) le long d'une deuxième ligne de coupe (101), dans lequel la deuxième ligne de coupe (101) couvre les parties latérales (20, 22) et un bord longitudinal respectif (91) de la bande de partie principale de couche (90), et dans lequel la deuxième ligne de coupe (101) croise la première ligne de coupe (100), de telle manière que la deuxième ligne de coupe (101) ne s'étende pas à travers la première ligne de pliage (36) des parties latérales antérieures et postérieures (20, 22).

2. Procédé selon la revendication 1, **caractérisé en ce que** la deuxième ligne de coupe (101) s'étend à partir d'un point A sur la première ligne de coupe (100) d'une partie latérale postérieure (20) suivant une courbe vers l'intérieur en direction de la région d'entrejambe (10) d'abord jusqu'à un point B d'un bord longitudinal postérieur respectif (41) de la partie principale (4) et ensuite dans la partie principale (4), dans lequel le point A est espacé de la première ligne de pliage (36) vers l'intérieur en direction transversale, et la ligne de coupe (101) s'étend ensuite à travers la région d'entrejambe (10) de la partie principale (4) puis en s'y raccordant en une courbe vers l'extérieur à travers un point C du bord latéral antérieur (42) de la partie principale (4) et enfin jusqu'à un point D sur la première ligne de coupe (100) des parties latérales antérieures (22), dans lequel le point D est espacé, vers l'intérieur en direction transversale, de la première ligne de pliage (36) des parties latérales antérieures (22).

3. Procédé selon la revendication 2, **caractérisé en ce que** le point A est espacé de 2-60 mm vers l'intérieur de la première ligne de pliage (36).

4. Procédé selon la revendication 3, **caractérisé en ce que** le point D est espacé de 2-60 mm vers l'intérieur de la première ligne de pliage (36).

5. Procédé selon l'une quelconque des revendications précédentes, **caractérisé en ce que** la bande de matériau plat (50) est pliée sur elle-même autour d'une deuxième ligne de pliage (37) s'étendant en direction longitudinale (28), dans lequel la deuxième ligne de pliage (37) est disposée plus près du bord latéral (70, 70a, 70b) que la première ligne de pliage (36).

6. Procédé selon l'une quelconque des revendications précédentes, **caractérisé en ce que** la bande de matériau plat (50, 50a) est pliée sur elle-même autour d'une troisième ligne de pliage (39) s'étendant en direction longitudinale (28), dans lequel la troisième ligne de pliage (39) est disposée plus près du bord latéral (70, 70a, 70b) que la deuxième ligne de pliage (37).

7. Procédé selon l'une quelconque des revendications précédentes, **caractérisé en ce que** les zones partielles pliées l'une sur l'autre de la bande de matériau plat (50, 50a) sont fixées de manière séparable.

8. Procédé selon l'une quelconque des revendications précédentes, **caractérisé en ce que** la bande de matériau plat (50a) est réalisée à double largeur, de telle manière que deux parties de matériau (66a) proviennent d'une partie longitudinale (57) de la bande de matériau plat (50a), dans lequel les premières découpes de matériau (52) et le pliage de la bande de matériau plat (50a) sont effectués de part et d'autre, donc sur un premier (70a) et sur un deuxième (70b) bord longitudinal et la bande de matériau plat (50a) est divisée dans la direction longitudinale.

9. Procédé selon la revendication 8, **caractérisé en ce que** la bande de matériau plat (50a) est divisée après la formation des premières découpes de matériau (52) et après le pliage de la bande de matériau plat (50a) dans la direction longitudinale (28).

10. Procédé selon l'une quelconque des revendications précédentes, **caractérisé en ce que** des moyens de fermeture (32) sont appliqués sur la bande de matériau plat (50, 50a).

11. Procédé selon l'une quelconque des revendications précédentes, **caractérisé en ce que** les couches jetables (2) sont fabriquées de telle manière que, lorsque des couches sont transportées en se suivant dans la direction longitudinale (28), la région dorsale (8) d'une couche se raccorde à la région dorsale (8) de l'autre couche et que la région antérieure (6) d'une couche se raccorde à la région antérieure (6) de l'autre couche.

12. Procédé selon l'une quelconque des revendications précédentes, **caractérisé en ce que** les couches jetables (2) sont fabriquées de telle manière que, lorsque des couches sont transportées en se suivant dans la direction longitudinale (28), la région dorsale (8) d'une couche se raccorde à la région antérieure (6) de l'autre couche et que la région antérieure (6) d'une couche se raccorde à la région dorsale (8) de l'autre couche.

13. Procédé selon l'une quelconque des revendications précédentes, **caractérisé en ce qu'**une partie de matériau (66, 66a) séparée transversalement à la direction longitudinale (28) forme des parties latérales (20, 22) de deux couches transportées l'une à la suite de l'autre.

14. Procédé selon l'une quelconque des revendications précédentes, **caractérisé en ce que** les parties latérales (20, 22) de la région antérieure (6) et de la région dorsale (8) sont réalisées différemment.

15. Procédé selon l'une quelconque des revendications précédentes, **caractérisé en ce que**, après l'étape de procédé consistant à fixer de manière inséparable les parties de matériau (66, 66a) à un bord longitudinal respectif (91) d'une bande de partie principale de couche (90), le rapport de la largeur maximale B1 de la bande de couche transportée dans la direction longitudinale à la largeur minimale B2 de la bande de couche dans la région d'entrejambe avant la formation des deuxièmes découpes de matériau vaut au minimum 1,1 et au maximum 2.
